# EUROPEAN PATENT APPLICATION

(11) **EP 4 837 960 A1**
(43) Date of publication of application: **11.11.2026**
(21) Application number: 24915499.8
(22) Date of filing: 26.12.2024
(51) Int. Cl.: C12N 5/02, C12N 1/04, C12N 5/09, C12N 9/99

(54) **MEDIUM FOR CULTURING AND/OR STORING CANCER CELLS, METHOD FOR PRIMARY CULTURE OF CANCER CELLS, METHOD FOR SUBCULTURE OF CANCER CELLS, AND METHOD FOR STORING CANCER CELLS**

(30) Priority: 04.01.2024 JP 2024000352
(71) Applicant: Fujita Academy, Toyoake-shi, Aichi 470-1192 (JP)
(72) Inventor: YAMAMOTO, Naoki, Toyoake-shi, Aichi 470-1192 (JP); HARA, Noriko, Toyoake-shi, Aichi 470-1192 (JP); IMAIZUMI, Kazuyoshi, Toyoake-shi, Aichi 470-1192 (JP); KONDO, Masashi, Toyoake-shi, Aichi 470-1192 (JP); HOSHIKAWA, Yasushi, Toyoake-shi, Aichi 470-1192 (JP)
(74) Representative: Heyerhoff Geiger GmbH & Co. KG
(86) International application number: PCT/JP2024/046241
(87) International publication number: WO 2025/146810

(57) **Abstract**

An object is to provide a culture medium suitable for primary culture of cancer cells. The object can be achieved by a culture medium for culture and/or preservation of cancer cells in which the culture medium contains at least a ROCK inhibitor and monothioglycerol.

## Description

### [Technical Field]

The disclosure of the present application relates to a culture medium for culture and/or preservation of cancer cells, a primary culture method of cancer cells, a subculture method of cancer cells, and a preservation method of cancer cells.

### [Background Art]

In general, established cancer cell lines are used for basic research on chemotherapy using anticancer agents or the like. However, cancer cell lines that have been continuously maintained and cultured in vitro for long years may be changed in nature from the original patient's tumor tissue and may not sufficiently reflect behavior in vivo. Accordingly, primary culture of cancer cells is considered promising for more accurate development of anticancer agents or optimal selection of treatment on a patient basis.

Several methods for primary culture are already known. For example, Patent Literature 1 discloses that, (1) to primarily culture cells in a tissue (biological tissue) collected from a living organism by using a culture medium used for typical cell culture without particularly adding any growth factors or inhibitors, (2) cells in a tissue collected from a living organism are seeded and cultured on the top face of a cell structure that includes cells making up the stroma and has a single cell layer or two or more cell layers laminated in the thickness direction.

Patent Literature 2 discloses a primary culture method that can mass-culture patient-derived cancer cells in vitro in a simplified manner, in a short time, and stably by (a) mincing a cancer tissue derived from a living organism into fragments and removing impurities from the minced cancer tissue, (b) providing a tissue clump obtained in step (a) for suspension culture, and then (c) providing the cultured product obtained in step (b) for adherent culture.

### [Citation List]

### [Patent Literature]

Patent Literature 1: International Publication No. 2019/039457
Patent Literature 2: Japanese Patent Application Laid-Open No. 2018-011576

### [Summary of Invention]

### [Technical Problem]

Primary culture of cancer cells from a cancer tissue of patients varies in the culture success rate in accordance with organs. In general, the success rate of primary culture of cancer cells from substantial organs, such as cells of colorectal cancer, gastric cancer, liver cancer, or the like is relatively high, but the primary culture is not always successful. In contrast, lung cancer is said to be one of the cancer types with which the primary culture of cancer cells thereof is the most difficult, and the success rate of primary culture of cancer cells from lung cancer tissues is generally said to be around 10% (Giard DJ, et al., "In vitro cultivation of human tumors: establishment of cell lines derived from a series of solid tumors.", J Natl Cancer Inst., 1973 Nov; 51(5): 1417-23. doi: 10. 1093/jnci/51. 5. 1417. PMID: 4357758).

In Patent Literatures 1 and 2, however, there is no description to the effect that the composition of a culture medium is studied with a focus on the success rate of primary culture. In the field of primary culture of cancer cells, there is a demand for providing a culture medium suitable for primary culture.

The disclosure of the present application has been made to solve the above problem. The present inventors have made an intensive study and thus newly found that primary culture of cancer cells can be suitably implemented by using a culture medium containing at least a ROCK inhibitor and monothioglycerol.

That is, the disclosure of the present application intends to provide a culture medium suitable for primary culture of cancer cells.

### [Solution to Problem]

The disclosure of the present application relates to a culture medium for culture and/or preservation of cancer cells, a primary culture method of cancer cells, a subculture method of cancer cells, and a preservation method of cancer cells illustrated below.

(1) A culture medium for culture and/or preservation of cancer cells, the culture medium including at least:
   a ROCK inhibitor and monothioglycerol.
(2) The culture medium according to (1) above, wherein the ROCK inhibitor is of at least one type selected from a group consisting of Y-27632 2HCl, Y-27632, H-1152 dihydrochloride, Hydroxyfasudil (HA-1100) HCl, GSK269962A HCl, Thiazovivin, Fasudil (HA-1077) HCl, GSK429286A, RKI-1447, Azaindole 1 (TC-S 7001), Y-39983 HCl, Netarsudil (AR-13324) 2HCl, Belumosudil (KD025), and AT13148.
(3) The culture medium according to (2) above, wherein the ROCK inhibitor is of at least one type selected from a group consisting of Y-27632 2HCl, Y-27632, H-1152 dihydrochloride, Hydroxyfasudil (HA-1100) HCl, and GSK269962A HCl.
(4) The culture medium according to (1) above,
   wherein the final concentration of the ROCK inhibitor in the culture medium is greater than or equal to 0.05 µmol/L and less than or equal to 100 µmol/L, and
   wherein the final concentration of the monothioglycerol in the culture medium is greater than or equal to 10 µmol/L and less than or equal to 5000 µmol/L.
(5) The culture medium according to (3) above,
   wherein the final concentration of the ROCK inhibitor in the culture medium is greater than or equal to 0.05 µmol/L and less than or equal to 100 µmol/L, and
   wherein the final concentration of the monothioglycerol in the culture medium is greater than or equal to 10 µmol/L and less than or equal to 5000 µmol/L.
(6) The culture medium according to (1) above further including an antibiotic and/or an antifungal agent.
(7) The culture medium according to (1) above, wherein the cancer is lung cancer.
(8) The culture medium according to any one of (1) to (7) above,
   wherein the culture medium is a culture medium for preservation of cancer cells, and
   wherein the cancer cells are preserved at normal temperature.
(9) A primary culture method of cancer cells, the primary culture method including:
   a culture step of culturing cancer cells collected from a living organism by using the culture medium according to any one of (1) to (7) above.
(10) A subculture method of cancer cells, the subculture method including:
   a passage step of, during passage, using a chelator on cancer cells cultured by the primary culture method according to (9) above to reduce a likelihood of adhesion of the cancer cells to a wall of a culture container, taking the cancer cells out of the culture container, and transferring the cancer cells to another culture container containing a fresh culture medium, the chelator binding to divalent metal ions.
(11) A preservation method of cancer cells, the preservation method including:
   a preservation step of immersing cancer cells collected from a living organism into the culture medium according to any one of (1) to (7) above and preserving the cancer cells at normal temperature.
(12) The preservation method according to (11) above, wherein the culture medium contains fetal bovine serum.

### [Advantageous Effects of Invention]

With the use of the culture medium disclosed in the present application, primary culture of cancer cells can be suitably implemented. Further, the culture medium disclosed in the present application can also be used as a culture medium for subculture or preservation of cancer cells.

### [Brief Description of Drawings]

[FIG. 1]

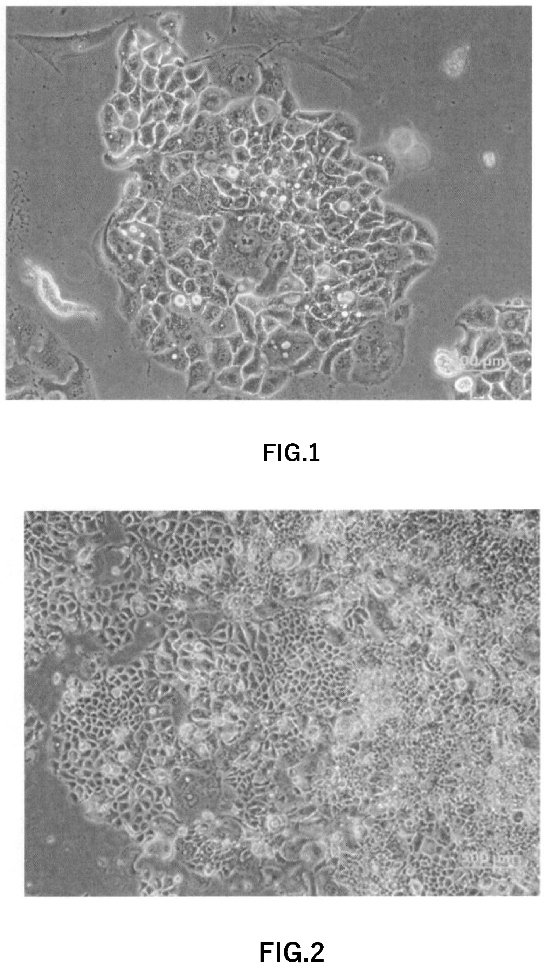

   FIG. 1 is a photograph substitute for a drawing, which is a photograph of lung cancer cells obtained by primary culture in Example 2.
[FIG. 2]
   FIG. 2 is a photograph substitute for a drawing, which is a photograph of the third passage of lung cancer cells obtained by subculture in Example 4.
[FIG. 3]

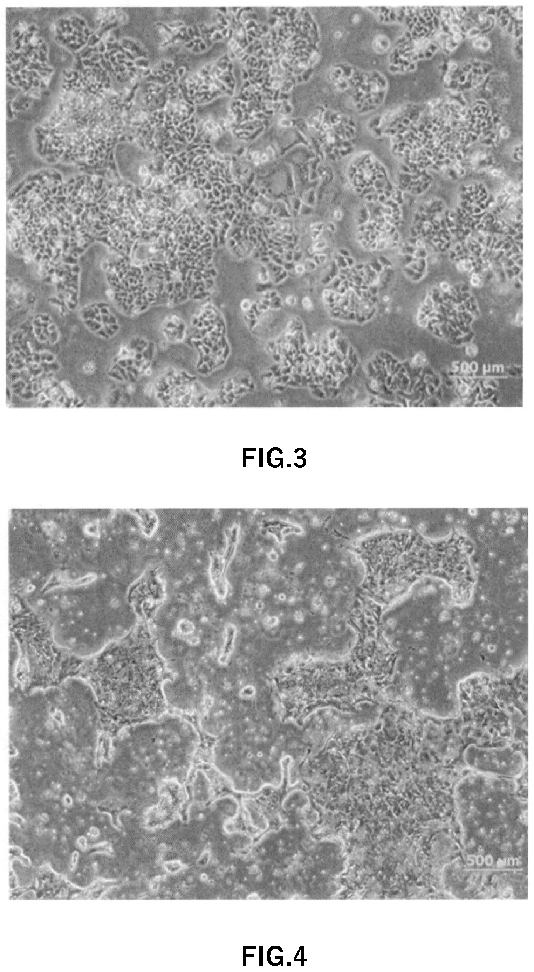

   FIG. 3 is a photograph substitute for a drawing, which is a photograph of the fourth passage of lung cancer cells obtained by subculture in Example 4.
[FIG. 4]
   FIG. 4 is a photograph substitute for a drawing, which is a photograph of lung cancer cells obtained by primary culture after preserved at normal temperature for three days in Example 6.
[FIG. 5]

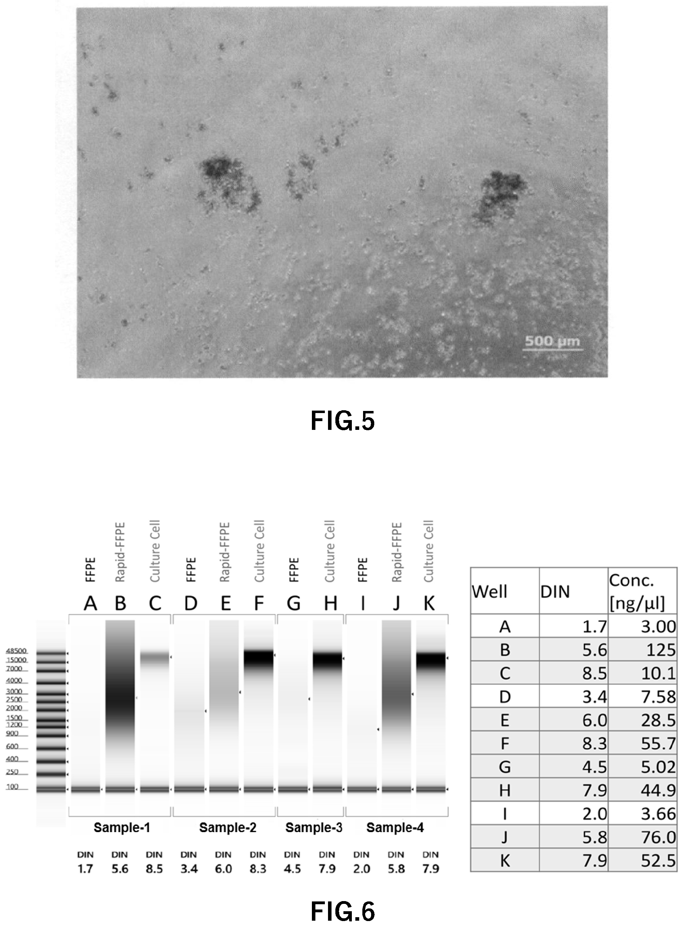

   FIG. 5 is a photograph substitute for a drawing, which is a photograph of lung cancer cells obtained by primary culture after preserved in a refrigerator for three days in Comparative example 4.
[FIG. 6]
   FIG. 6 is an electrophoresis photograph illustrating a result of examination of qualities of DNA extracted from the formalin-fixed paraffin-embedded (FFPE), DNA extracted from the Rapid-FFPE, and DNA extracted from Culture Cell in Example 7.

### [Description of Embodiments]

A culture medium for culture and/or preservation of cancer cells (hereafter, which may be simply referred to as "culture medium"), a primary culture method of cancer cells (hereafter, which may be simply referred to as "primary culture method"), a subculture method of cancer cells (hereafter, which may be simply referred to as "subculture method"), and a preservation method of cancer cells (hereafter, which may be simply referred to as "preservation method") disclosed in the present application will be described below in detail.

Further, in the present specification, it is construed that:
(1) numerical ranges expressed by using "to" mean a range including numerical values stated before and after "to" as the lower limit value and the upper limit value, respectively;
(2) numerical values, numerical ranges, and qualitative expressions (for example, expressions such as "identical", "same", or the like) represent the numerical values, numerical ranges, and properties including errors generally accepted in this technical field; and
(3) when "substantially XX shape" is stated, a shape recognized as not only accurately XX shape but also approximately XX shape is included.

### [Embodiment of culture medium]

The culture medium according to an embodiment contains at least a ROCK inhibitor and monothioglycerol.

The ROCK (Rho kinase; Rho-associated protein kinase) is a serine-threonine protein kinase identified as a target protein of the low-molecular-weight GTP-binding protein Rho and is involved in various physiological functions, such as smooth muscle contraction and cellular morphological change. The ROCK inhibitor is a compound that inhibits activity of the above ROCK. The ROCK inhibitor is not particularly limited as long as it can inhibit activity of the ROCK. Commercially available ROCK inhibitors may be, but not limited to, Y-27632 2HCl, Y-27632, H-1152 dihydrochloride, Hydroxyfasudil (HA-1100) HCl, GSK269962A HCl, Thiazovivin, Fasudil (HA-1077) HCl, GSK429286A, RKI-1447, Azaindole 1 (TC-S 7001), Y-39983 HCl, Netarsudil (AR-13324) 2HCl, Belumosudil (KD025), AT13148, or the like. The ROCK inhibitor contained in the culture medium may be composed of one type of the above or may be composed of two or more types of the above in combination.

The ROCK inhibitors listed above as examples are compounds illustrated below, respectively.

| | |
|---|---|
| Y-27632 2HCl | 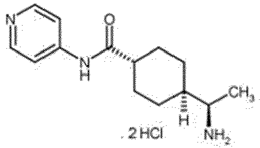 |
| Formula (1) | |
| Y-27632 | 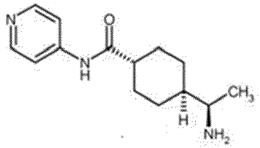 |
| Formula (2) | |
| H-1152 dihydrochloride | 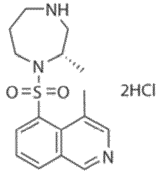 |
| Formula (3) | |
| Hydroxyfasudil (HA-1100) HCl | 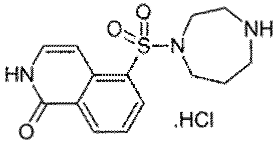 |
| Formula (4) | |
| GSK269962A HCl | 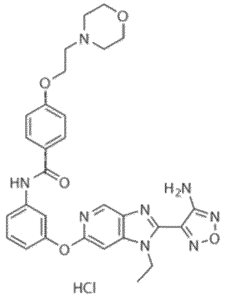 |
| Formula (5) | |
| Thiazovivin | 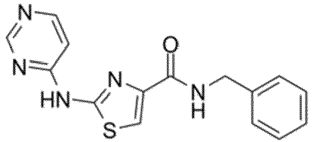 |
| Formula (6) | |
| Fasudil (HA-1 077) HCl | 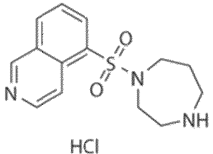 |
| Formula (7) | |
| GSK429286A | 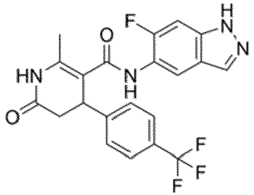 |
| Formula (8) | |
| RKI-1447 | 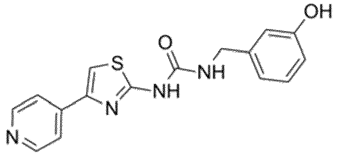 |
| Formula (9) | |
| Azaindole 1 (TC-S 7001) | 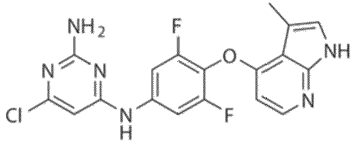 |
| Formula (10) | |
| Y-39983 HCl | 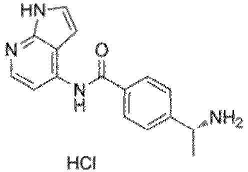 |
| Formula (11) | |
| Netarsudil (A R-13324) 2HCl | 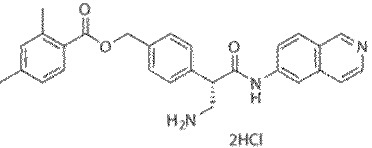 |
| Formula (12) | |
| Belumosudil (KD025) | 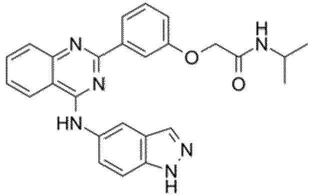 |
| Formula (13) | |
| AT13148 | 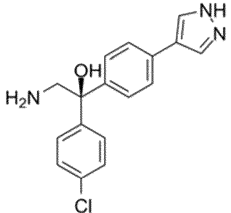 |
| Formula (14) | |

Among the ROCK inhibitors listed above as examples, Y-27632 2HCl, Y-27632, H-1152 dihydrochloride, Hydroxyfasudil (HA-1100) HCl, or GSK269962A HCl are more preferable.

The monothioglycerol is a compound expressed by the following formula (15), which is a compound used for applications of stabilizers or antioxidants for antibiotics, curling agents (perm solutions) because the excellent reducing ability thereof helps cleave S-S bonds in hair, antimicrobial preservatives, or the like.

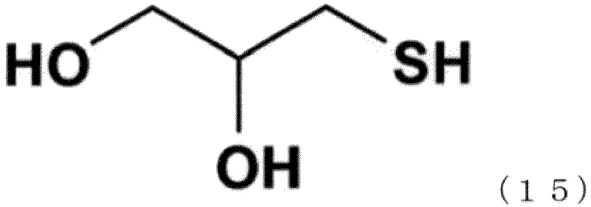

For the culture medium, known culture media for cell culture can be used as the basic culture medium except for the requirement of inclusion of the ROCK inhibitor and monothioglycerol as essential components. The culture medium may be, but not limited to, DMEM (Dulbecco's Modified Eagle's Medium), DMEM: F-12 (Dulbecco's Modified Eagle Medium: Nutrient Mixture F-12), EMEM (Eagle's minimal essential medium), MEMα (Minimum Essential Medium Alpha), BME (Basal Medium Eagle), RPMI-1640, or the like. For these culture media, culture media to which various components such as cell growth factors are added, if necessary, to adjust the composition in accordance with cell types can be used.

Various components typically added to culture media may be, for example, cell growth factors such as FGF-2 (Fibroblast Growth Factor-2), TGF-β (Transforming Growth Factor-β), EGF (Epidermal Growth Factor), VEGF (Vascular Endothelial Growth Factor), or the like; vitamins or vitamin derivatives such as ascorbic acid, retinoic acid, or the like; sugar sources such as glucose; amino acid; inorganic salts such as sodium selenite, sodium chloride, or the like; proteins such as transferrin; hormone such as insulin; differentiation-inhibiting factors; differentiation-inducing factors such as dexamethasone, oncostatin M, or the like; antioxidants such as 2-mercaptoethanol, dithiothreitol, or the like; or the like.

Further, for some types of cancer (in particular, gastric cancer, colorectal cancer), direct culturing of cells collected from a patient may cause proliferation of bacteria or fungi attached to the collected cancer cells. Accordingly, an antibiotic and/or an antifungal agent may be added to the culture medium if necessary. The antibiotic may be, but not limited to, penicillin, streptomycin, gentamicin, or the like, and the antifungal agent may be, but not limited to, amphotericin B, fluconazole, or the like.

The cancer may be of any type. The examples of cancer may be, for example, but not limited to, colorectal cancer, small intestine cancer, gastric cancer, esophageal cancer, anal cancer, pancreatic cancer, liver cancer, bile duct cancer, gastrointestinal neuroendocrine tumor, gastrointestinal stromal tumor (GIST), breast cancer, lung cancer, mesothelioma, thymic carcinoma, kidney cancer, urothelial carcinoma, testicular tumor, prostate cancer, endometrial cancer, cervical cancer, uterine sarcoma, malignant ovarian tumor, tongue cancer, gingival cancer, floor of mouth cancer, pharyngeal cancer, laryngeal cancer, salivary gland cancer, thyroid cancer, osteosarcoma, Ewing sarcoma, soft tissue sarcoma, myelodysplastic syndrome, skin cancer, neuroblastoma, malignant glioma (glioblastoma), malignant lymphoma, multiple myeloma, or the like.

The culture medium according to the embodiment has achieved primary culture at a high success rate not only for gastric cancer, liver cancer, and pancreatic cancer but also for lung cancer with which primary culture of cancer cells thereof has conventionally been difficult, as illustrated in Examples described below.

The concentrations of the ROCK inhibitor and monothioglycerol added to the culture medium are not particularly limited as long as it is within a range that enables primary culture of cancer cells to be suitably implemented. The final concentration of the ROCK inhibitor in the culture medium may be, for the lower limit value, but not limited to, 0.05 µmol or higher, 0.06 µmol or higher, 0.07 µmol or higher, 0.08 µmol or higher, 0.1 µmol or higher, 0.2 µmol or higher, 0.4 µmol or higher, 0.6 µmol or higher, 0.8 µmol or higher, 1 µmol or higher, 2 µmol or higher, 3 µmol or higher, 4 µmol or higher, 5 µmol or higher, 6 µmol or higher, 7 µmol or higher, 8 µmol or higher, 9 µmol or higher, 10 µmol or higher, or the like per 1 L of the culture medium and may be, for the upper limit value, but not limited to, 100 µmol or lower, 90 µmol or lower, 80 µmol or lower, 70 µmol or lower, 60 µmol or lower, 50 µmol or lower, or the like per 1 L of the culture medium.

The final concentration of monothioglycerol in the culture medium may be, for the lower limit value, but not limited to 10 µmol or higher, 20 µmol or higher, 30 µmol or higher, 40 µmol or higher, 50 µmol or higher, 60 µmol or higher, 70 µmol or higher, 80 µmol or higher, 90 µmol or higher, 100 µmol or higher, or the like per 1 L of the culture medium and may be, for the upper limit value, but not limited to, 5000 µmol or lower, 4000 µmol or lower, 3000 µmol or lower, 2000 µmol or lower, 1000 µmol or lower, 750 µmol or lower, 500 µmol or lower, or the like per 1 L of the culture medium.

Further, the culture medium according to the embodiment can be suitably used not only in primary culture but also in subculture or preservation of cancer cells collected from patients, as illustrated in Examples described below. In particular, cancer cells with the culture medium according to the embodiment can be preserved at normal temperature without requiring refrigeration. Therefore, convenience in handling of collected cancer cells is improved. Note that, in the present specification, "normal temperature" means a temperature for preservation without use of any cooling element or heating element, which means about 15 °C to 30 °C though varying with seasons. Further, in the present specification, "preservation" means keeping collected cancer cells alive while suppressing death of the cancer cells as much as possible during a period from collection of cancer cells from a living organism to implementation of a primary culture method (preservation period). Note that cancer cells during a preservation period may be left to stand in the same place or may be transported (for example, may be transported from a hospital where cancer cells are collected to a facility where primary culture is implemented thereon).

The culture medium disclosed in the present application achieves the following advantageous effects.
(1) The culture medium disclosed in the present application can increase the success rate of primary culture of lung cancer cells for which primary culture has conventionally been difficult and, in addition, can be used for primary culture of cancer cells of cancer other than lung cancer. Therefore, since primary culture of cancer cells can be performed regardless of types of cancer, the culture medium disclosed in the present application is useful for clinical applications of genetic analysis for diagnosis and treatment, selection of an anticancer agent, or the like and for basic research on molecular mechanisms of cancer, genetic mutation analysis, drug discovery research, or the like.
(2) The culture medium disclosed in the present application can be used as a culture medium for preservation of cancer cells in addition to primary culture and subculture. Therefore, since even one type of culture medium can be used for different applications, the culture medium disclosed in the present application is useful in practical sites such as clinical settings.

### [Embodiment of primary culture method]

Next, the primary culture method according to the embodiment will be described. The primary culture method includes a culture step of culturing collected cancer cells by using the culture medium of any of those described in the embodiment of the culture medium described above.

The subject from which cancer cells are to be collected may be any living organisms (living animals), preferably, human having cancer. Cancer tissues of living organisms can be collected by known methods. The methods may be, but not limited to, collection or excision during surgery, biopsy, or the like.

The culture method in the culture step is not particularly limited as long as cancer cells collected from a living organism can be cultured. Typically, a tissue containing cancer cells collected from a living organism is subjected to mincing before cultured. In the mincing, a knife, scissors, or the like can be used to mince the tissue into fragments to the degree that the tissue clump is no longer visible to the naked eye (about 1 mm square or less). Further, the culture method may be explant culture to directly culture tissues containing minced cancer cells or may be enzymatic dissociation culture to dissociate tissues containing minced cancer cells into cell units by enzyme and then culture the cells. Note that, in the present specification, the reference to "culture cancer cells" represents a concept including both culturing a tissue containing minced cancer cells and culturing cancer cells dissociated into cell units. Further, in the present specification, the reference to "cancer cell(s)" includes both "isolated cancer cell(s)" and "a tissue containing cancer cell(s)". The enzyme used for enzymatic dissociation culture is not particularly limited as long as it is an enzyme typically used in the field of cell culture and may be, but not limited to, collagenase, trypsin, papain, dispase, or the like.

Further, if necessary, cancer cells may be sieved by using cleaned and sterilized filter or the like, and thereby impurities can be removed.

As known culture methods of cancer cells, there are adherent culture that causes cancer cells to grow in a monolayer on an artificial substrate and suspension culture that causes cancer cells to suspend freely and grow in a culture medium. While the adherent culture is suitable for most cell types including primary culture, the suspension culture is suitable mainly for non-adhesive cells. Therefore, while the adherent culture is represented as a preferable example in the primary culture method according to the embodiment, cancer cells may be initially cultured by suspension culture to form a cell aggregation and then be subjected to adherent culture.

For the culture container used in the culture step, known container typically used in the field of cell culture can be used. For the adherent culture, culture containers whose surface has been treated to facilitate cells or cell clumps to adhere thereto are commercially available, and these culture containers may be used, but is not limited thereto. For some types of cancer or tissue types, cancer cells may be seeded on a plate immobilized with laminin 511, laminin 411, soluble E-cadherin, or the like. Further, the shape or the size of the culture container can also be selected in accordance with conditions such as types of cancer cells or required volumes. Since various sizes of flasks, bottles, dishes, tubes, plates, or the like are commercially available, these can be used.

In suspension culture, for the culture container used for the suspension culture, culture containers whose surface has been treated to make it difficult for cells or cell clumps to adhere thereto are commercially available, and these culture containers may be used. Further, the shape or the size of the culture container can also be selected in accordance with conditions such as types of cancer cells or required volumes. Since various sizes of flasks, bottles, dishes, tubes, plates, or the like are commercially available, these can be used.

The culture conditions such as a culture time, a temperature, or the like can be determined as appropriate in accordance with types of cancer by a person who implements culture.

The primary culture method according to the embodiment achieves the following advantageous effects.
(1) The product of the primary culture from cancer cells that have proliferated in the culture step is similar to a collected clinical tissue of a living organism. That is, the state of a cancer tissue in a living organism can be reproduced in vitro. Thus, the product of the primary culture obtained by the primary culture method according to the embodiment can be used for elucidation of cancer molecular mechanisms, drug sensitivity testing, screening of medicines suitable for the collected biological samples (patients), genetic analysis of cancer cells, or the like. Further, the resultant of the primary culture can also be used for research and development such as drug discovery.
(2) The use of the culture medium disclosed in the present application improves the success rate of lung cancer cells, primary culture of which has been difficult, to around 70%. Note that, in the present specification, "success" of "success rate" means that cancer cells are increased by around 100 times by primary culture. The improved success rate increases the probability of successfully implementing elucidation of cancer molecular mechanisms, drug sensitivity testing, screening of medicines suitable for the collected biological samples (patients), genetic analysis of cancer cells, or the like as described above in (1). Therefore, tailor-made medical treatment specific to each patient can be realized.
(3) Cancer tissues collected during surgery or the like are typically subjected to formalin fixation, and a paraffin block thereof is prepared and used for pathological observation or the like. Since the formalin used in tissue fixation induces crosslinking between nucleic acids (such as DNA) and proteins, DNA is more likely to be subjected to a physical stress. Further, formalin may be oxidized into formic acid, and the formic acid causes DNA to depurinate and leads to DNA strand breakage. Therefore, even if DNA analysis were attempted by using cancer tissues fixed with formalin, there would be a problem of a significantly poor DNA quality. In contrast, since cancer cells obtained by the primary culture method allows for high-quality gDNA to be obtained as illustrated in Examples described below, the accuracy of genetic analysis is improved.

### [Embodiment of subculture method]

Next, the subculture method according to the embodiment will be described. The subculture method includes a passage step of, during passage, using a chelator on cancer cells cultured by the primary culture method described above to reduce the likelihood of adhesion of the cancer cells to a wall of a culture container, taking the cancer cells out of the culture container, and transferring the cancer cells to another culture container containing a fresh culture medium, in which the chelator binds to divalent metal ions.

The culture medium used in the subculture method can be the same as the culture medium used in the primary culture method. Alternatively, the culture medium component used in the subculture method may differ from the culture medium component used in the primary culture method as long as they contain the ROCK inhibitor and monothioglycerol. Further, while subculture implemented after the passage step is preferably adherent culture, there is no problem even with suspension culture. The passage step and the subculture may be repeated multiple times.

The chelator is not particularly limited as long as it binds to divalent metal ions in the culture medium (calcium ions, zinc ions, copper ions, magnesium ions, iron ions, or the like). The chelator may be, but not limited to, ethylenediaminetetraacetic acid (EDTA), ethylene glycol tetraacetic acid (EGTA), 1,2-bis(o-aminophenoxy)ethane-N,N,N',N'-tetraacetic acid (BAPTA), or the like.

The concentration of the chelator added during passage is not particularly limited as long as it is within a range that enables taking out of cultured cells from the culture container and does not cause significant damage to these cells. A chelator solution at 1 mM can be added by about 0.5 to 1 mL, but is not limited thereto.

In the conventional cell culture method, enzyme is used to peel cells from a culture container during passage. However, the use of enzyme makes cell membranes or receptors of cell membrane surfaces vulnerable, and weakened cells may reduce the proliferative capability thereof. Further, receptors or the like poorly expressed on the cell membrane surface may disappear and may be undetectable during cell membrane surface analysis. In contrast, in the subculture method according to the embodiment, the use of a chelator that binds to divalent metal ions makes it possible to easily take cancer cells out of a culture container. In other words, it can be said that the passage step is a step of taking cancer cells out of a culture container without using enzyme and transferring the cancer cells to another culture container containing a fresh culture medium.

With the subculture method according to the embodiment, an advantageous effect of improved efficiency of subculture can be obtained.

### [Embodiment of preservation method]

Next, the preservation method according to the embodiment will be described. The preservation method according to the embodiment includes a preservation step of immersing cancer cells collected from a living organism into the culture medium described in the embodiment of the culture medium and preserving the cancer cells at normal temperature.

The culture medium has been described in the above embodiment of the culture medium, and the cancer cells collected from a living organism have been described in the above embodiment of the primary culture method. Thus, detailed description thereof will be omitted to avoid duplicated description.

Preservation of cancer cells in a refrigerator will cause damage to the cells. Thus, even when primary culture is performed on cancer cells preserved in a refrigerator, the cancer cells do not proliferate in some cases. In contrast, the preservation method according to the embodiment uses the culture medium disclosed in the present application to enable the preservation at normal temperature. Therefore, an advantageous effect of improved convenience in handling of cells in performing primary culture on cancer cells collected by surgery can be obtained. Further, since cancer cells can be preserved at normal temperature, for example, since cancer cells collected at a clinic that does not have cell culture facilities can be transported at normal temperature to a facility capable of primary culture, an advantageous effect of improved convenience in transport of cancer cells can be obtained.

The preservation period is not particularly limited as long as it is within a range that does not cause death of cancer cells during preservation or does not cause an extreme reduction in proliferation capability and is preferably about one to four days. Further, optionally and additionally, antibiotics such as penicillin, streptomycin, or gentamicin may be added to the culture medium.

When collected cancer cells are preserved by using a preservation culture medium for a long time, it is desirable that fetal bovine serum (FBS) be contained in the preservation culture medium. Further, it is desirable that serum substitutes such as KSR (serum-free supplement) be also contained in addition to FBS. Containing FBS (and serum-free supplement) results in stable preservation of cancer cells. The concentration of FBS contained in the preservation culture medium may be, for the lower limit value, but not limited to, 0.5% by volume or higher, 0.75% by volume or higher, 1.0% by volume or higher, 1.5% by volume or higher, 2.0% by volume or higher, 2.5% by volume or higher, 3.0% by volume or higher, or the like and may be, for the upper limit value, but not limited to, 20% by volume or lower, 18% by volume or lower, 16% by volume or lower, 14% by volume or lower, 12% by volume or lower, 10% by volume or lower, or the like.

The concentration of the serum-free supplement may be, for the lower limit value, but not limited to, 0.5% by volume or higher, 0.75% by volume or higher, 1.0% by volume or higher, 1.5% by volume or higher, 2.0% by volume or higher, 2.5% by volume or higher, 3.0% by volume or higher, or the like and may be, for the upper limit value, but not limited to, 20% by volume or lower, 18% by volume or lower, 16% by volume or lower, 14% by volume or lower, 12% by volume or lower, 10% by volume or lower, or the like.

Note that, when an animal-derived component is contained in the preservation culture medium, viruses or the like may be contaminated into cultured cancer cells. Even when viruses are contaminated, there is no particular problem when cultured cancer cells are used for research and development application or screening in drug discovery or the like. In terms of further enhancement of safety, however, it is desirable that no FBS (and serum-free supplement) be contained in the preservation culture medium for a case of a short preservation period of collected cancer cells. Note that, in the present specification, "long-term preservation" means preservation for three or more days, and the short term means preservation for less than three days.

Although Examples are presented below to specifically describe the embodiment disclosed in the present application, these Examples are provided merely to illustrate the embodiment. These Examples express neither limitation nor restriction of the technical scope disclosed in the present application.

### [EXAMPLES]

### [Preparation of culture medium]

### <Example 1>

A culture medium was prepared with the following composition.
1. Culture media for primary culture and for subculture (common to both adherent culture and suspension culture) and a culture medium for preservation (for short-term preservation)
   - 1 µM A83-01 (TGF-β inhibitor; manufactured by FUJIFILM Wako Pure Chemical Corporation)
   - 50 ng/mL EGF (epidermal growth factor; manufactured by Sigma-Aldrich)
   - 2 mM CHIR99021 (GSK-3 inhibitor; manufactured by FUJIFILM Wako Pure Chemical Corporation)
   - 1 µmol/L Y-27632 (ROCK inhibitor; manufactured by FUJIFILM Wako Pure Chemical Corporation)
   - x100 N2 supplement (manufactured by Thermo Fisher Scientific)
   - x50 B27 supplement included (manufactured by Thermo Fisher Scientific)
   - 20 ng/mL bFGF (basic fibroblast growth factor; manufactured by Sigma-Aldrich)
   - 2 mM Glutamax^{™} Supplement (glutamic acid; manufactured by Thermo Fisher Scientific)
   - 50 µmol/L monothioglycerol (manufactured by FUJIFILM Wako Pure Chemical Corporation)
   - x100 MEM Non-Essential Amino Acids Solution (manufactured by Thermo Fisher Scientific)
   - 0.5 mg/mL BSA (bovine serum albumin; manufactured by FUJIFILM Wako Pure Chemical Corporation)
   - 100 units/mL penicillin G (manufactured by FUJIFILM Wako Pure Chemical Corporation)
   - 100 µg/mL streptomycin sulfate (manufactured by FUJIFILM Wako Pure Chemical Corporation)
2. Culture medium for long-term preservation
   - 5% by volume FBS (fetal bovine serum; manufactured by Thermo Fisher Scientific)
   - 2.5% by volume KSR (serum-free supplement; manufactured by Thermo Fisher Scientific)
   - 1 mmol/L sodium pyruvate (manufactured by Thermo Fisher Scientific)
   - 2.5% by volume human serum (manufactured by Sigma-Aldrich)
   - 2 mM Glutamax^{™} Supplement (glutamic acid; manufactured by Thermo Fisher Scientific)
   - 1 µmol/L Y-27632 (ROCK inhibitor; manufactured by FUJIFILM Wako Pure Chemical Corporation)
   - 50 ng/mL SAG (Smoothened agonist; manufactured by Abcam)
   - 50 µmol/L monothioglycerol (manufactured by FUJIFILM Wako Pure Chemical Corporation)
   - RPMI 1640 medium (manufactured by Thermo Fisher Scientific)
   - 100 units/mL penicillin G (manufactured by FUJIFILM Wako Pure Chemical Corporation)
   - 100 µg/mL streptomycin sulfate (manufactured by FUJIFILM Wako Pure Chemical Corporation)

### [Implementation of primary culture]

### <Example 2>

### 1. Culture container

In Example 2, the following culture container was used.
- Multi-well plate (1/12 well) for adherent culture. Note that the multi-well plate for adherent culture was coated overnight in a refrigerator by using iMatrix-511 coating (manufactured by Takara Bio).
   Culture medium used for culture: 1 mL/well.
- HydroCell plate (24 well) for suspension culture. Note that the plate for suspension culture was not coated by iMatrix-511 coating (manufactured by Takara Bio).
   Culture medium used for culture: 1 mL/well.

### 2. Procedure of primary culture

(1) A lung cancer tissue collected by surgery was put into 15 mL tube containing 2 mL of the culture medium for short-term preservation and preserved for 2 hours.
(2) The tissue was transferred to a 10-cm dish by using a wide-bore tip, and a small amount of the culture medium for short-term preservation was added thereto. The tissue was finely minced by using a scalpel blade (into about 0.5 to 1 mm square of fragments). The finely minced tissue strip was put into the culture medium for short-term preservation again and incubated at 37 °C for 30 minutes.
(3) The solution was made up to 10 mL with PBS, and centrifugal washing (1,000 rpm, 5 min) was performed thereon.
(4) The supernatant was aspirated and removed, and the solution was subjected to enzymatic treatment. Specifically, 2 mL of 0.1% Collagenase I was added to the solution and, after Voltex, the solution was subjected to enzymatic reaction (37 °C, 20 to 30 min, shaking). Note that 0.1% Collagenase I was obtained by diluting Collagenase I (SIGMA) 0.5% with HBSS (containing Mg, Ca) by 5 times.
(5) The solution was made up to 10 mL with 9 mL of PBS + 1 mL of CELLOTION (manufactured by Takara Bio), and centrifugal washing (1,000 rpm, 5 min) was performed thereon.
(6) 2 mL of PBS was added to the product, and the tissue was dissociated by pipetting. The resulting solution of 2 mL was subjected to filtration into a new 15 mL tube together with a tissue residue, the tissue residue left on the filter was lightly ground by a pipette, and the tube was then washed with about 7 mL of PBS, and the wash was subjected to filtration. Then, 1 mL of CELLOTION was added thereto, and centrifugal washing (1,000 rpm, 5 min) was performed thereon.
(7) The supernatant was aspired and removed as much as possible, and a hemolytic agent was added to the product, and hemolytic treatment (4 °C, 15 min) was performed after Voltex.
(8) The solution was made up to 10 mL with PBS and 1 mL of CELLOTION, and centrifugal washing (1,000 rpm, 5 min) was performed thereon.
(9) The supernatant was aspired and removed, the product was made up to 10 mL with PBS and 1 mL of CELLOTION again, and centrifugal washing (1,000 rpm, 5 min) was performed thereon. Note that 12 µL of cell suspension was loaded onto a counting chamber before centrifuged, and the number of cells was counted.
(10) The supernatant was aspired and removed up to 100 µL, and cancer cells were seeded into the culture medium for primary culture (the well-plate for adherent culture and the plate for suspension culture described in the above section "1. Culture container") (the number of seeded cancer cells were determined as appropriate).
(11) The cells were cultured for about 7 days in a cell culturing incubator at 37 °C with 5% carbon dioxide. Note that the culture medium was replaced about once every two to three days.

Note that, in Example 2, lung cancer tissues collected from 62 patients (N = 62) were divided into two halves, which were subjected to the experiment with adherent culture and suspension culture, respectively. In other words, inspection was performed by two culture methods of adherent culture and suspension culture on the samples from the same cases. The results were that 30 cases were successful in primary culture with both the adherent culture and the suspension culture, 4 cases were successful in primary culture with only the adherent culture, and 12 cases were successful in primary culture with only the suspension culture. That is, 46 out of 62 cases were successful in primary culture (the number of lung cancer cells increased by 100 times or more) with both or any one of the adherent culture and the suspension culture, that is, about 74% of the lung cancer tissues collected from living organisms were successful in primary culture. FIG. 1 represents a photograph of lung cancer cells obtained by primary culture (adherent culture).

### <Comparative example 1>

An experiment was conducted in the same procedure as in Example 2 except that the ROCK inhibitor was not added in the culture medium composition of Example 1 (N = 11), and almost no cancer cells proliferated with both the adherent culture and the suspension culture.

### <Comparative example 2>

An experiment was conducted in the same manner as in Example 2 except that monothioglycerol was not added in the culture medium composition of Example 1 (N = 8), and the cancer cells decreased by 20% within one week with both the adherent culture and the suspension culture.

From the results of Example 2 and Comparative examples 1 and 2, it has been confirmed that, with the use of the culture medium to which the ROCK inhibitor and monothioglycerol are added in combination, primary culture can be performed at a high success rate even for lung cancer cells with which the successful rate of primary culture is said to be significantly low. Further, it has been confirmed that the culture medium disclosed in the present application is useful for both adherent culture and suspension culture.

### <Example 3>

Primary culture was performed in the same procedure as in Example 2 except that, instead of lung cancer tissues, cancer tissues collected during surgery of osteosarcoma, gastric cancer, ovarian cancer, colorectal cancer, pancreatic cancer, and esophageal cancer were used (however, for gastric cancer and colorectal cancer, 2.5 µg/mL amphotericin B (manufactured by FUJIFILM Wako Pure Chemical Corporation) and 5 µg/mL fluconazole (manufactured by FUJIFILM Wako Pure Chemical Corporation) were added). The success rates of the primary culture (the number of cells increased by 100 times or more) were as follows. Note that, although the primary culture was successful with both the adherent culture and the suspension culture, unlike Example 2, only the results of culture methods exhibiting high success rates are illustrated.
Osteosarcoma: 3 out of 4 cases were successful. The success rate was 75%.
Gastric cancer: 7 out of 10 cases were successful. The success rate was 70%. Note that, although the primary culture of the 3 failed cases was stopped because of contamination of fungi, proliferation of cancer cells was confirmed.
Ovarian cancer: 2 out of 3 cases were successful. The success rate was 67%.
Colorectal cancer: 4 out of 5 cases were successful. The success rate was 80%.
Pancreatic cancer: 6 out of 10 cases were successful. The success rate was 60%. Note that, for 3 out of 4 failed cases, it was separately confirmed that no cancer cell was contained in the resected tissues.
Esophageal cancer: 2 out of 2 cases were successful. The success rate was 100%.

It has been confirmed from the above results that the culture medium disclosed in the present application is useful for primary culture not only for lung cancer cells but also for many other types of cancer cells. In particular, the success rate of the primary culture of lung cancer cells is drastically improved to about 74% with the use of the culture medium disclosed in the present application while the success rate was reported to be about 10% with the conventional method.

### [Implementation of subculture]

### <Example 4>

Following the primary culture of Example 2 described above, subculture was performed in the following procedure. Notet that lung cancer cells resulted from primary culture with the adherent culture were used for the subculture. The passage step is illustrated as follows.
(1) The culture medium was aspired, and lung cancer cells were washed with PBS.
(2) As the chelator, 1 mL of a 1 mM EDTA solution was added, the lung cancer cells were reacted for 5 to 15 minutes in a carbon dioxide incubator at 37 °C and observed by a microscope every 5 minutes. When peeling of cells from the dish started, PBS was added to perform pipetting, and the cells were harvested.
(3) The cells were transferred to a 15 mL tube, made up to 10 mL with PBS and 1 mL of CELLOTION, and centrifugal washing (1,000 rpm, 5 min) was performed thereon.

Further subculture was performed on some of the subcultured second-passage lung cancer cells. FIG. 2 represents a photograph of the third passage, and FIG. 3 represents a photograph of the fourth passage. Note that the success rate of subculture from the second passage to the fourth passage was about 90%.

### <Comparative example 3>

Subculture was performed in the same procedure as in Example 4 except that, instead of the chelator of Example 4, enzyme (0.05% Trypsin, 1 mL) was added. The result was that most cancer cells died, and the passaging failed at a frequency of 50% to 70%.

### [Implementation of primary culture and subculture by using other ROCK inhibitors]

### <Example 5>

Primary culture was performed in the same procedure as in Example 2 except that, instead of Y-27632 of Example 2, four agents of Y-27632 2HCl (manufactured by MedChemExpress), H-1152 dihydrochloride (manufactured by MedChemExpress), Hydroxyfasudil (HA-1100) HCl (manufactured by MedChemExpress), and GSK269962A HCl (manufactured by MedChemExpress) were used as the ROCK inhibitors. The success rates of the primary culture were as follows. Note that, although the primary culture was successful with both the adherent culture and the suspension culture, unlike Example 2, only the results of culture methods exhibiting high success rates are illustrated.
- Y-27632 2HCL: 2 out of 3 cases were successful. The success rate was 67%.
- H-1152 dihydrochloride: 3 out of 4 cases were successful. The success rate was 75%.
- Hydroxyfasudil (HA-1100) HCl: 3 out of 4 cases were successful. The success rate was 75%.
- GSK269962A HCl: 2 out of 3 cases were successful. The success rate was 67%.

Further, from lung cancer cells, pancreatic cancer cells, and gastric cancer cells cultured up to the second passage by using four types of ROCK inhibitors of Example 5, lung cancer cells (three cases), pancreatic cancer cells (two cases), and gastric cancer cells (three cases) were selected and further subcultured, all of which were able to be passaged up to the fourth passage. Based on Y-27632 2HCl as a reference (defined as 1), the number of proliferations of cells by passage was 0.99 for H-1152 dihydrochloride, 1.13 for Hydroxyfasudil (HA-1100) HCl, and 1.03 for GSK269962A HCl, which represents similar proliferation.

It has been confirmed from the above results that the use of the culture medium disclosed in the present application enables primary culture and subculture at high success rates regardless of types of cancer cells.

### [Culture medium for preservation]

### <Example 6, Comparative example 4>

Lung cancer cells collected from living organisms were immersed in the culture medium for long-term preservation, preserved at normal temperature (Example 6) and in a refrigerator (Comparative example 4) for three days, and then primary culture (however, only the adherent culture) was performed thereon in the same procedure as in Example 2. FIG. 4 represents a photograph of lung cancer cells preserved at normal temperature on the seventh day from the primary culture. FIG. 5 represents a photograph of lung cancer cells preserved in the refrigerator on the seventh day from the primary culture. As is clear from the photograph of FIG. 4, it has been confirmed that the cancer cells proliferate even when the cancer cells collected from living organisms are preserved at normal temperature in the culture medium for long-term preservation. In contrast, as is clear from the photograph of FIG. 5, when the cancer cells collected from living organisms are preserved in the refrigerator, less proliferation of the cancer cells was observed compared to the case of preservation at normal temperature. In general, when cancer cells collected from living organisms are preserved in a refrigerator when not immediately cultured. Since the use of the culture medium disclosed in the present application enables preservation at normal temperature of collected cancer cells, convenience in handling of collected cells is improved.

### <Comparative example 5>

An experiment was conducted in the same procedure as in Example 6 except that a culture medium excluding the ROCK inhibitor and monothioglycerol from the culture medium used in Example 2, that is, a conventionally, typically used culture medium was used. Note that, in Comparative example 5, the experiment was conducted on ovarian cancer cells in addition to lung cancer cells. The result was as follows.
- Lung cancer: 0 out of 3 cases was successful. The success rate was 0%.
- Ovarian cancer: 0 out of 2 cases was successful. The success rate was 0%.

It has been confirmed from the above results that the culture medium disclosed in the present application can be used for any application of primary culture, subculture, and preservation. Furthermore, it has been confirmed that preservation at normal temperature is more preferable when cancer cells are preserved.

### [DNA quality]

### <Example 7>

Next, an experiment for examining the DNA quality of cancer cells obtained by primary culture was conducted. The experimental procedure is illustrated below.

### <Preparation of samples>

### (1) DNA from formalin-fixed paraffin-embedded (FFPE)

The lung cancer tissues collected during surgery from four patients (denoted as Samples 1, 2, 3, 4, respectively, in Fig. 4 illustrating the result) were subjected to formalin fixation by typical procedure and treatment time and then cut, and a paraffin block for pathological diagnosis was prepared (formalin-fixed paraffin-embedded (FFPE)) by a typical process. From paraffin sections prepared by a typical scheme by using microtome from the above block, genome DNA (gDNA) was extracted by using commercially available DNA extraction kit.

### (2) DNA from Rapid-FFPE

The lung cancer tissues collected during surgery from the three patients described above (without Sample 3) were immediately cut into a size of about 5 to 10 mm, and a formalin-fixed paraffin block was prepared by a typical method. Subsequently, a paraffin section was prepared in the same manner as FFPE described above, and gDNA extraction was performed.

### (3) DNA from Culture Cell

The lung cancer tissues collected during surgery from the four patients described above were put into the culture medium for short-term preservation and transported to a cell culture room. Primary culture was then performed in accordance with the procedure of Example 2. Then, gDNA was extracted from cancer cells that have proliferated by culture.

### <Electrophoresis>

The quality of gDNA extracted in the above section <Preparation of sample> was examined by using TapeStation system (by Agilent). FIG. 6 illustrates the result. As illustrated in the result of gel electrophoresis of FIG. 6, almost no band was seen in FFPE. On the other hand, a band was seen but was broad in Rapid-FFPE. This is considered to be because proteins and DNA were crosslinked and DNA degradation occurred due to formalin used for tissue fixation. On the other hand, DNA was not degraded, and a clear band was observed in the primary culture (Culture Cell). The value of DNA Integrity Number (DIN) in determination of a relative quality of genome DNA (gDNA) of each sample was the highest for Culture Cell as is clear from FIG. 6. Note that the evaluation range of DIN is 1 to 10, and a higher DIN value indicates less degradation of gDNA.

From the above results, since high quality gDNA can be obtained from cancer cells resulted from primary culture, the gDNA can also be used for genetic analysis on a patient basis or the like.

### [Industrial Applicability]

The use of the culture medium disclosed in the present application enables primary culture of cancer cells at a high success rate. Therefore, the culture medium disclosed in the present application is useful for the medical industry.

## Claims

1. A culture medium for culture and/or preservation of cancer cells, the culture medium comprising at least:
a ROCK inhibitor and monothioglycerol.

2. The culture medium according to claim 1, wherein the ROCK inhibitor is of at least one type selected from a group consisting of Y-27632 2HCl, Y-27632, H-1152 dihydrochloride, Hydroxyfasudil (HA-1100) HCl, GSK269962A HCl, Thiazovivin, Fasudil (HA-1077) HCl, GSK429286A, RKI-1447, Azaindole 1 (TC-S 7001), Y-39983 HCl, Netarsudil (AR-13324) 2HCl, Belumosudil (KD025), and AT13148.

3. The culture medium according to claim 2, wherein the ROCK inhibitor is of at least one type selected from a group consisting of Y-27632 2HCl, Y-27632, H-1152 dihydrochloride, Hydroxyfasudil (HA-1100) HCl, and GSK269962A HCl.

4. The culture medium according to claim 1,
wherein the final concentration of the ROCK inhibitor in the culture medium is greater than or equal to 0.05 µmol/L and less than or equal to 100 µmol/L, and
wherein the final concentration of the monothioglycerol in the culture medium is greater than or equal to 10 µmol/L and less than or equal to 5000 µmol/L.

5. The culture medium according to claim 3,
wherein the final concentration of the ROCK inhibitor in the culture medium is greater than or equal to 0.05 µmol/L and less than or equal to 100 µmol/L, and
wherein the final concentration of the monothioglycerol in the culture medium is greater than or equal to 10 µmol/L and less than or equal to 5000 µmol/L.

6. The culture medium according to claim 1 further comprising an antibiotic and/or an antifungal agent.

7. The culture medium according to claim 1, wherein the cancer is lung cancer.

8. The culture medium according to any one of claims 1 to 7,
wherein the culture medium is a culture medium for preservation of cancer cells, and
wherein the cancer cells are preserved at normal temperature.

9. A primary culture method of cancer cells, the primary culture method comprising:
a culture step of culturing cancer cells collected from a living organism by using the culture medium according to any one of claims 1 to 7.

10. A subculture method of cancer cells, the subculture method comprising:
a passage step of, during passage, using a chelator on cancer cells cultured by the primary culture method according to claim 9 to reduce a likelihood of adhesion of the cancer cells to a wall of a culture container, taking the cancer cells out of the culture container, and transferring the cancer cells to another culture container containing a fresh culture medium, the chelator binding to divalent metal ions.

11. A preservation method of cancer cells, the preservation method comprising:
a preservation step of immersing cancer cells collected from a living organism into the culture medium according to any one of claims 1 to 7 and preserving the cancer cells at normal temperature.

12. The preservation method according to claim 11, where in the culture medium contains fetal bovine serum.
